Europäisches Patentamt

European Patent Office

Office européen des brevets

(11) Veröffentlichungsnummer: **0 327 803**
**A2**

## EUROPÄISCHE PATENTANMELDUNG

(21) Anmeldenummer: 89100194.3

(22) Anmeldetag: 07.01.89

(51) Int. Cl.⁴: **A61M 35/00**

(30) Priorität: 09.02.88 DE 3803838

(43) Veröffentlichungstag der Anmeldung:
16.08.89 Patentblatt 89/33

(84) Benannte Vertragsstaaten:
**BE CH DE ES FR GB IT LI LU NL**

(71) Anmelder: **GEORG KARL GEKA-BRUSH GMBH**

**D-8809 Bechhofen-Waizendorf(DE)**

(72) Erfinder: **Fitjer, Holger**
**Lambrechtstr. 15**
**D-8800 Ansbach(DE)**

(74) Vertreter: **Czowalla . Matschkur Patentanwälte**
**Dr.-Kurt-Schumacher-Strasse 23 Postfach**
**9109**
**D-8500 Nürnberg 11(DE)**

(54) **Einmal-Applikationsgerät.**

(57) Einmal-Applikationsgerät mit einem den Halteschaft eines Wegwerf-Applikators aufnehmenden stilförmigen Haltegriff, mit vorzugsweise abgewinkeltenm Kopfende, insbesondere für Kosmetikanwendungen, Dentallabors, Zahnärzte od.dgl., wobei der
Haltegriff und der Wegwerf-Applikator so ausgebildet
sind, daß der Applikator berührungsfrei am Haltegriff
befestigt und wieder gelöst werden kann.

FIG. 1

EP 0 327 803 A2

## Einmal-Applikationsgerät

Die Erfindung bezieht sich auf ein Einmal-Applikationsgerät mit einem den Halteschaft eines Wegwerf-Applikators aufnehmenden stilförmigen Haltegriff mit vorzugsweise abgewinkelten Kopfende, insbesondere für Kosmetikanwendungen, Dentallabors oder Zahnärzte.

Sowohl beim Auftragen von flüssigen, pulverförmigen oder postösen kosmetischen Mitteln als auch bei der Zahnbehandlung oder bei den Arbeiten in Dentallabors, aber auch in vielen anderen technischen Laboranwendungen, ergibt sich immer wieder die Notwendigkeit einen Applikator, dessen Kopfende als Bürstchen, Pinsel, Tampon od.dgl. ausgebildet sein kann, zu verwenden, wobei es entscheidend darauf ankommt, daß äußerst genau hygienische Bedingungen eingehalten werden und andererseits auch jede Gefahr einer Verschmutzung oder Gesundheitsbeeinträchtigung des mit dem Gerät Arbeitenden ausgeschlossen ist. Zu diesem Zweck sind bisher meist derartige Einmal-Applikationsgeräte so ausgebildet, daß der Wegwerf-Applikator mit einem einfachen Haltegriff von vornherein vereinigt in einer Verpackung untergebracht ist, so daß nach der Anwendung Applikator mit Griff weggeworfen werden muß.

Um diese Materialverschwendung zu vermeiden ist erfindungsgemäß bei einem Einmal-Applikationsgerät aus einem stilförmigen Haltegriff und einem getrennten Wegwerf-Applikator vorgesehen, daß der Haltergriff und der Wegwerf-Applikator so ausgebildet sind, daß der Applikator berührungsfrei am Haltegriff befestigt und wieder gelöst werden kann, so daß der Benutzer der Applikationsgeräte weder zum Einsetzen diesen in die Hand nehmen muß, noch es notwendig ist daß er ihn in Hand nimmt um ihn wieder aus dem Haltegriff zu entfernen.

Gemäß seiner ersten Ausführungsform der vorliegenden Erfindung ist zu diesem Zweck vorgesehen, daß der Haltegriff zumindest im vorderen Abschnitt gabelförmig derart ausgebildet ist, daß die Gabelarme mit Haltezapfen von außen in, vorzugsweise als Durchgangsbohrungen ausgebildete, Vertiefungen des Halteschafts des Wegwerf-Applikators eingreifen, bzw. umgekehrt und daß zur Erzielung einer verkippsicheren Halterung ineinandergreifende Nut- und Federabschnitte des Haltegriffs und des Halteschafts des Wegwerf-Applikators vorgesehen sind. Diese Nut- und Federabschnitte können dabei besonders einfach in der Weise ausgebildet sein, daß entweder die Vertiefungen in zum freien Ende offenen Nuten des Halteschlitzes des Applikators angeordnet sind, die der Breite der die Zapfen tragenden Klemmenden des Haltegriffs entsprechen, oder daß umgekehrt, falls Zapfen und Vertiefungen vertauscht angebracht sind, entsprechende Längsnuten in den Klemmenden des Haltegriffs vorgesehen sind, welche den Halteschaft entsprechend verkippsicher aufnehmen können.

Die Gabelarme können entweder in der Weise federnd ausgebildet sein, daß sie - in der Ruhestellung elastisch federnd aneinanderliegend, von außen federnd in die Vertiefungen eingreifen oder aber die Federung kann auch so vorgesehen sein, daß die Gabelarme normalerweise in der Öffnungsstellung sind und von außen in die Vertiefungen eingedrückt werden, wobei in diesem Fall eine lösbare Verriegelungseinrichtung für die in Klemmstellung gegeneinandergedrückten Gabelarme vorgesehen sein muß.

Diese Verriegelungsvorrichtung kann in Ausgestaltung der Erfindung entweder in der Weise erzielt werden, daß die Gabelarme auf der Innenseite mit verriegelnd ineinandersprengbaren Rasten und Gegenrasten, also beispielsweise Zapfen und Vertiefungen, versehen sind, oder aber auch indem ein längs der nach hinten keilförmig sich erweiternden Gabelschenkel arretierbar verschiebbarer Klemmring od.dgl. vorgesehen ist. Beim Aufnehmen eines Wegwerf-Applikators wird der Klemmring nach hinten verschoben, wodurch die federnden Gabelarme zusammengedrückt werden und den Halteschaft festklemmend ergreifen können. Zum Wiederauswerfen des Wegwerf-Applikators bedarf es lediglich einer Verschiebung des Klemmrings nach vorne, wobei durch die Verjüngung der Außenflächen aufeinander zu die federnden Gabelarme selbsttätig auseinander federn und damit den Wegwerf-Applikator freigeben.

Welche der möglichen Klemmprinzipien man anwendet ist dabei im wesentlichen eine Geschmacksfrage, da jedes dieser Prinzipien Vorteile entweder bei der Aufnahme, oder aber beim Wiederauswerfen des Applikators besitzt.

Darüber hinaus läßt sich das erfindungsgemäße Prinzip des berührungsfreien Aufnehmens und Wiederauswerfens eines Wegwerf-Applikators am Haltegriff gemäß einer zweiten Ausführungsform der Erfindung auch dadurch erzielen, daß das Kopfende des Schiebers eine nach oben offene Bohrung zur Aufnahme des Halteschafts des Applikators aufweist, der mit einer vorzugsweise durchgehenden Ausnehmung für eine längs des Haltegriffs verschiebbar gelagerten Arretierschieber versehen ist.

Das Vorsehen eines derartigen Arretierschiebers ermöglicht dabei eine klemmfreie Aufnahme des Halteschafts in der Bohrung, da ja eine Klemmhalterung in der Praxis deshalb unbrauchbar ist, da sie nicht die erfindungsgemäß gewünschte berührungsfreie Aufnahme und das ebenso berührungsfreie Wiederauswerfen ermöglichen würde. Zwar wäre es noch denkbar, einen Applikator berührungsfrei klemmend in die Bohrung des Haltegriffs einzubringen, doch wäre es dann auf jeden Fall notwendig mit der Hand, oder aber mit einem sonstigen Werkzeug, nachzuhelfen, um den Applikator wieder aus dem Klemmsitz herausziehen zu können. Dies soll aber ja erfindungsgemäß gerade vermieden werden.

Bevorzugt soll die Ausbildung dabei so getroffen sein, daß der Halteschaft gegenüber der Bohrung verlängert nach oben aus dieser herausragt, so daß notfalls, falls er Applikator aufgrund von Fertigungstoleranzen einmal nicht frei in der Bohrung gehaltert ist, so daß er nach zurückziehen des Schiebers von alleine wieder nach unten herausfällt, von oben durch Druck auf den durch das Applikationsmittel ja nicht betroffenen Endabschnitt des Halteschafts herausgedrückt werden könnte.

Um die Wegwerf-Applikatoren besonders einfach aufnehmen zu können, ist neben der selbstverständlich auch möglichen losen Verpackung einer Vielzahl solcher Applikatoren in Weiterbildung der Erfindung vorgesehen, daß die Wegwerf-Applikatoren stehend mit nach oben gerichteten Halteschäften in vorzugsweise einzeln freilegbaren Vertiefungen einer Tiefziehfolie gelagert sind. Durch Abziehen beispielsweise der Folienabdeckung wird einer dieser nach oben stehender Halteschäfte freigelegt, so daß daran ein Haltegriff angesetzt werden kann mit dem dann der Wegwerf-Applikator aus der Vertiefung herausgehoben wird.

Eine weitere sehr vorteilhafte Möglichkeit einer hygienischen und dennoch einen guten Zugriff gestattenden Verpackung einer Vielzahl von Wegwerf-Applikatoren ergibt sich in weiterer Ausbildung der Erfindung dadurch, daß die Wegwerf-Aplikatoren mit frei längs einer Kante nach außen ragenden Halteschäften derart in Blisterstreifen verpackt sind, daß die geschützt zwischen den Folien des Blisterstreifens untergebrachten Applikatorköpfe nach Verbindung mit dem Haltegriff unter Aufreißen der Kante nach außen herausziehbar sind.

Weitere Vorteile, Merkmale und Einzelheiten der Erfindung ergeben sich aus der nachfolgenden Beschreibung einiger Ausführungsbeispiele, sowie anhand der Zeiohnung. Dabei zeigen:

Fig. 1-3 die Aufnahme, Arretierung und das Wiederauswerfen eines Wegwerf-Applikators an einem Haltegriff mit einem Verriegelungsschieber,

Fig. 4 eine teilweise geschnittene Seitensicht des in den Fig. 1 bis 3 verwendeten Haltegriffs,

Fig. 5 eine Aufsicht auf den Haltegriff nach Fig. 4,

Fig. 6 eine Ansicht des Haltegriffs in Richtung des Pfeils VI in Fig. 4,

Fig. 7 einen Schnitt längs der Linie VII-VII in Fig. 4,

Fig. 8 eine perspektivische Ansicht eines als U-förmige Zange ausgebildeten Haltegriffs mit federnden Gabelarmen,

Fig. 9 unterschiedliche Wegwerf-Applikatoren mit einem Halteschaft zur Verbindung mit einem Haltegriff gemäß Fig. 8,

Fig. 10 einen Schnitt durch das Kopfende des Halteschafts nach Fig. 8 mit einem darin gehalterten Wegwerf-Applikator,

Fig. 11 einen der Fig. 10 entsprechenden um 90˚ versetzten Schnitt,

Fig. 12 eine abgewandelte Ausführungsform eines gabelförmigen Haltegriffs in Seitenansicht,

Fig. 13 eine Aufsicht auf den Haltegriff nach Fig. 12,

Fig. 14 einen Längsschnitt durch das geöffnete Kopfende des Haltegriffs mit dazwischen angeordnetem Wegwerf-Applikator,

Fig. 15 einen Schnitt längs der Linie XV-XV in Fig. 14,

Fig. 16 eine schematische Aufsicht auf eine Verpakkung eines erfindungsgemäßen Einmal-Applikationsgeräts mit einem Haltegriff und einer Vielzahl von griffbereit in einer Verpackungsschale angeordneten Wegwerf-Applikatoren und

Fig. 17 eine Blisterstreifenverpackung für Wegwerf-Aplikatoren.

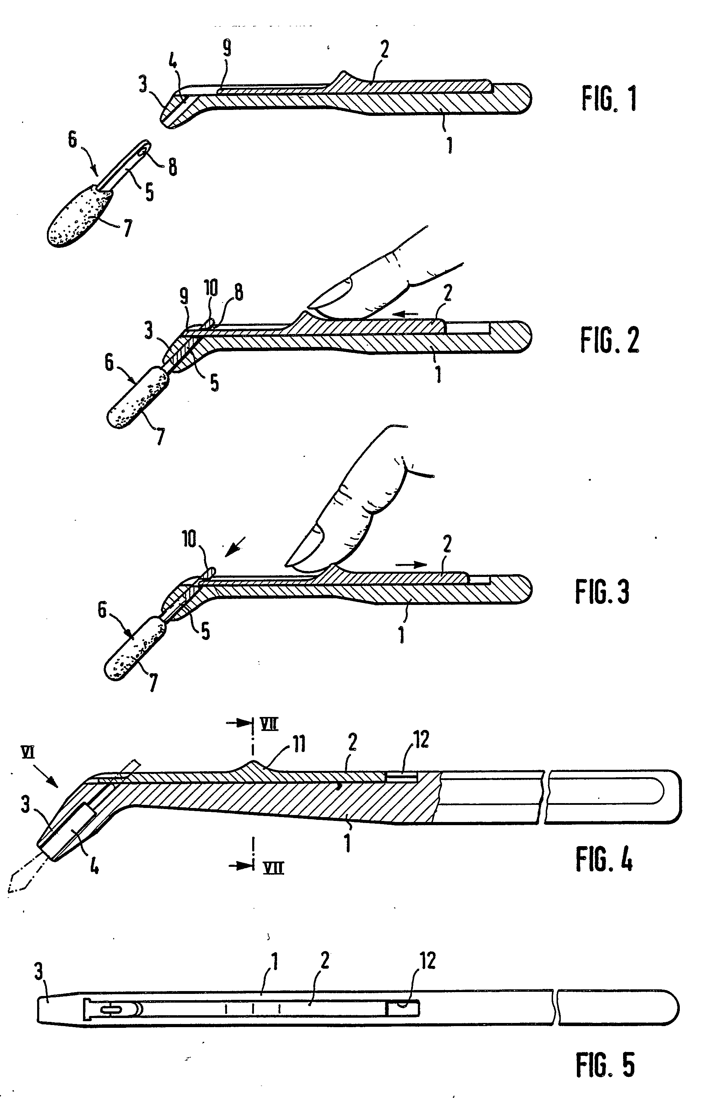

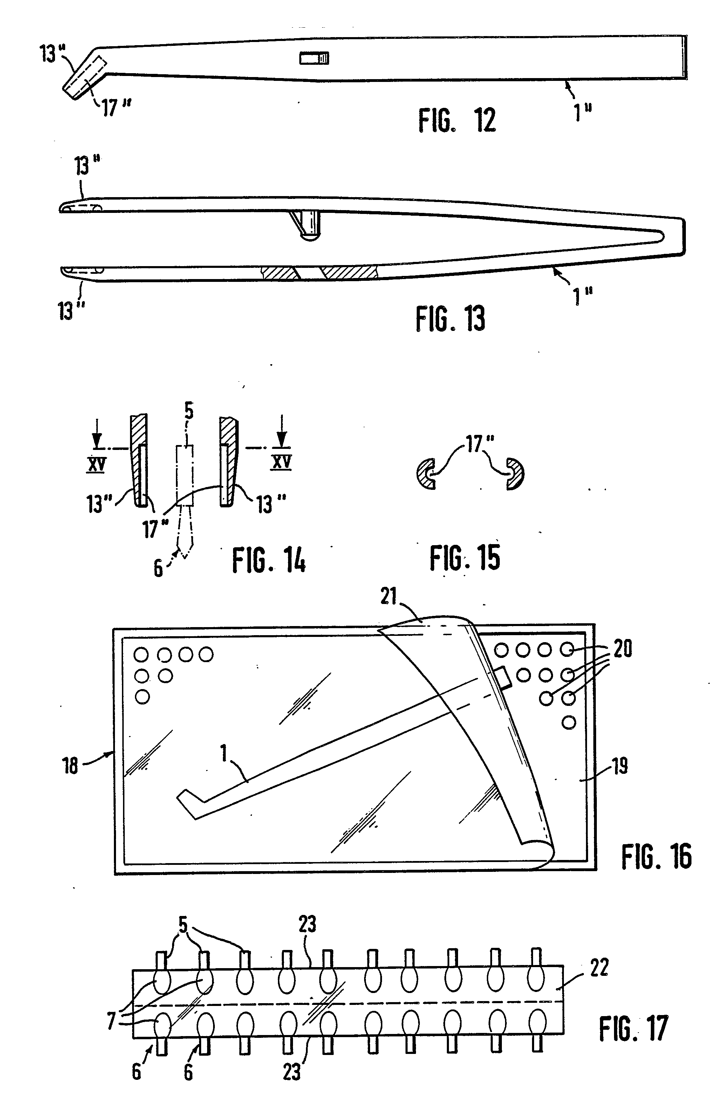

Anhand der Fig. 1 bis 3 ist das erfindungsgemäße Prinzip der berührungsfreien Befestigung und Wiederlösung von Wegwerf-Applikatoren an einem Haltegriff am Beispiels eines Haltegriffs 1 dargestellt, der mit einem Arretierschieber 2 versehen ist. Im vorderen abgewinkelten Kopfabschnitt des Schiebers ist eine durchgehende Bohrung 4 für den Halteschaft 5 eines Wegwerf-Applikators 6 vorgesehen. Im dargestellten Ausführungsbeispiel ist der Wegwerf-Applikator 6 mit einem als Tampon ausgebildeten Applikatorkopf 7 versehen. Der Halteschaft 5 des Wegwerf-Applikators 6 ist mit einer durchgehenden Ausnehmung 8 versehen, in welche die Spitze 9 eines verschiebbar auf der Oberseite des Haltegriffs 1 gelagerten Schiebers 2 eingreifen kann. Der beispielsweise stehend in einer napfförmigen Vertiefung einer Verpackungseinheit (vgl. hierzu die Fig. 15 und 16) angeordnete Applikator 6 wird durch Aufstecken der Bohrung 4 auf den Halteschaft 5 und anschließendes Nachvorneschieben des Schiebers 2 fest mit dem Haltegriff 1 verbunden. Damit der Schieber auch tatsächlich in die Bohrung 8 eindringen kann und diese nicht erst mühsam aufeinander ausgerichtet werden müssen, ist es vorteilhaft den Schaft querschnittlich ebenso unrund, beispielsweise quadratisch, auszusgestalten wie die Bohrung 4 des Haltegriffs 1. Nach dem Nachvorneschieben des Arretierschiebers 2 (vgl.

hierzu Fig. 2) ist der Wegwerf-Applikator 6 am Haltegriff 1 fest arretiert, so daß er für eine kosmetische Behandlung, eine Zahnbehandlung oder auch für sonstige Laborzwecke eingesetzt werden kann. Nach der Benutzung kann er berührungsfrei wieder ausgeworfen werden, indem lediglich der Schieber 2 nach rückwärts geschoben wird (Fig. 3), so daß der frei, d. h. nicht klemmend in der Bohrung 4 angeordnete Halteschaft 5 des Applikators entriegelt wird und der ganze Applikator somit nach unten herausfallen kann. Lediglich unter ungünstigen Umständen kann es einmal vorkommen, daß eine Verklemmung des Halteschafts 5 in der Bohrung 4 stattfindet. In diesem Fall läßt sich auch ohne Berührung des Applikatorkopfs 7 der Applikator herausdrücken, indem lediglich ein leichter Druck auf das nach oben über den Haltegriff überstehende Ende 10 des entsprechend verlängerten Halteschafts 5 ausgeliefert.

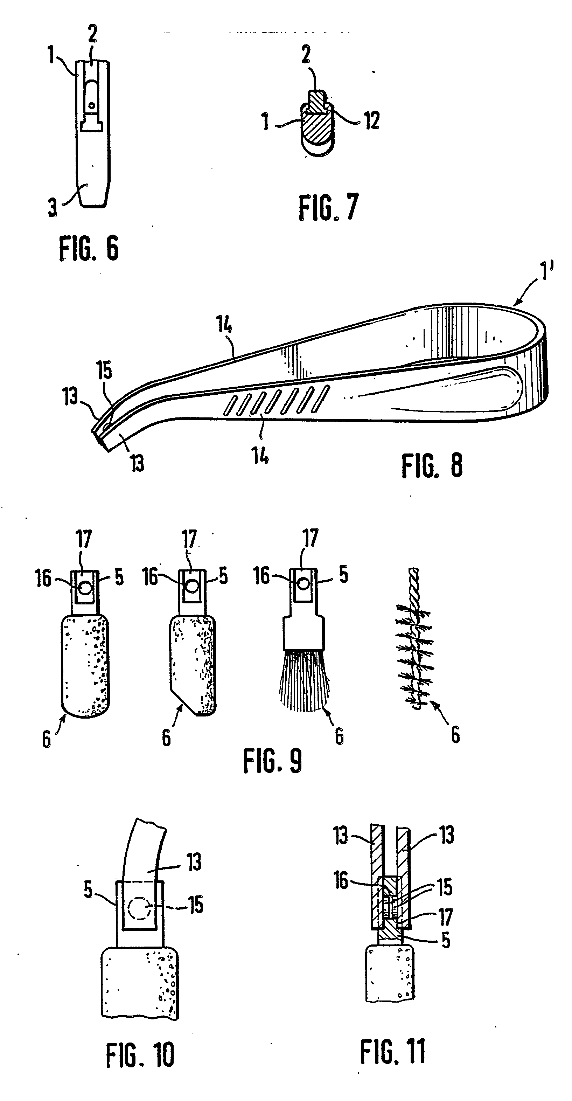

Aus den Fig. 4 bis 7 erkennt man im einzelnen den Aufbau des vorstehend in seiner Funktion beschriebenen Halteschafts mit einem Arretierschieber der zum vereinfachten Vordrücken und Zurückziehen mit einer Daumenbetätigungswulst 11 versehen ist. Der Schieber selbst ist, was man besonders gut aus Fig. 7 erkennen kann in einer hinterschnittenen Nut 12 auf der Oberseite des Schiebers 1 gelagert. Die Aufnahmebohrung 4 für den Halteschaft 5 des Applikators 6 ist bei dem in den Fig. 4 bis 7 dargestellten Haltegriff dabei abgesetzt ausgebildet, wobei der Halteschaft dann entsprechend ebenfalls abgesetzt sein muß, damit sein dünneres freies Ende aus dem verjüngten oberen Abschnitt der Bohrung herausstehen kann. Diese Ausführungsform ist für Applikatoren gedacht, bei denen nicht eine Anschlagschulter zwischen dem vorderen Ende des Haltegriffs und dem entsprechend verbreiterten Applikator vorgesehen ist, wie es beispielsweise bei einem schmalen Pinsel der Fall ist, der aus diesem Grund auch in Fig. 4 schematisch strichliert mit dargestellt ist.

Die Fig. 8 bis 11 zeigen eine erste Ausführungsform eines gabelförmig ausgebildeten Haltegriffs zum erfindungsgemäßen berührungsfreien Aufnehmen und Wiederauswerfen von Wegwerf-Applikatoren. Der Haltegriff 1′ ist dabei eine im wesentlich U-förmige Zange aus federelastischem Material, also beispielsweise Metall oder aber auch Kunststoff, dessen in ähnlicher Weise wie beim Arretierschieberhaltegriff nach den Fig. 1 bis 7 abgewinkeltes Kopfende ebenfalls abgewinkelt ist. Die abgewinkelten Klemmenden 13 der beiden Gabelarme 14 sind mit nach innen gerichteten Zapfen 15 versehen, welche in, vorzugsweise als durchgehende Bohrung ausgebildete Vertiefungen 16 des Halteschafts 5 eines Applikators 6 eingreifen. In Fig. 9 sind dabei vier verschiedene Typen von Applikatoren dargestellt, von denen der linke einen Schaumstoffkopf oder eine PU-Spitze aufweist, der zweite Kopf von links einen beflockten Spatel oder eine Füllspitze besitzt, der dritte von links eine Pinselspitze und der rechte schließlich eine gedrehte Bürste darstellt. Damit eine verkippungsfreie Halterung des Applikators durch den Haltegriff 1 erzielt wird, ist im Ausführungsbeispiels nach den Fig. 8 bis 11 der Halteschaft 5 jeweils auf einander gegenüberliegenden Seiten mit zum freien Ende des Halteschafts hin offenen Nuten 17 versehen, deren Breite der Breite der Klemmenden 13 der Gabelarme 14 entspricht, so daß durch die Einlagerung der Klemmarme in diesen Nuten eine verkipp- und verschwenkfreie Ausrichtung der Achsen der Klemmarme und der Achse des Wegwerf-Applikators erreicht wird. Die Ausbildung des Haltegriffs 1′ kann dabei so getroffen sein, daß die Gabelarme 14 aufeinander zufedern, so daß sie in der Ruhestellung ohne Applikator aneinanderliegen würden. Durch Aufweiten, d. h. beispielsweise durch Eingreifen mit dem Daumen zwischen die Gabelarme 14 werden die Klemmenden 13 geöffnet, so daß sie dann durch von außen in die Nuten 17 und die Vertiefungen 16 einfedern können. Statt dessen wäre selbstverständlich auch eine Ausbildung möglich, bei der die Gabelarme 14 normalerweise federnd in die Öffnungsstellung verspannt sind und somit entgegen ihrer Federwirkung zusammengedrückt werden müssen, damit sie in die Vertiefungen 16, 17 der Wegwerf-Applikatoren eingreifen. In diesem Fall müssen dann selbstverständlich Arretiermittel vorgesehen sein, um die Gabelarme 14 in dieser zusammengedrückten Stellung entgegen der nach außen wirkenden Federkraft zu halten. Dies könnten entweder Zapfen und Vertiefungen auf der Innenseite der Gabelarme sein, die beim Zusammendrücken rastend inneinandergreifen. Statt dessen könnte auch ein Klemmring vorgesehen sein, der zur Erzielung der den Wegwerf-Applikator haltenden Klemmstellung auf den Gabelarmen 14 nach hinten verschoben, so daß er infolge des sich konisch Öffnens der Gabelschenkel, deren vordere Klemmenden zusammendrückt. Beim Wieder-nach-vorne-Schieben des Klemmrings federn dann die Klemmenden selbsttätig nach außen auf und geben den Applikator frei. Um ein selbsttätiges oder ungewolltes Nach-vorne-Rutschen eines solchen Klemmrings zu verhindern, könnten beispielsweise Arretiernoppen auf der Außenseite der Gabelschenkel 14 vorgesehen sein. Die Fig. 12 bis 15 zeigen eine abgewandelte Ausführungsform, bei der nicht der Halteschaft 5 des Applikators 6 mit Nutausnehmungen für die Klemmenden des Haltegriffs 1″ versehen ist, sondern umgekehrt Nuten in den Klemmenden 13″ vorgesehen sind, welche den Halteschaft 5 aufnehmen. Die Nuten sind in diesem Fall mit 17″ bezeichnet.

Bei allen Anordnungen versteht es sich von selbst, daß es nicht erforderlich ist, die Zapfen an der Innenseite der Klemmenden 13 und die zugeordneten Vertiefungen 16 am Halteschaft 5 vorzusehen, sondern daß die Anordnung auch umgekehrt getroffen werden könnte.

In Fig. 16 ist eine Verpackungseinheit für eine erfindungsgemäßes Einmal-Applikationsgerät dargestellt, welches aus einer vorzugsweise wegen des Tiefziehens geformten Unterschale 19 mit einer Vielzahl von Einzelvertiefungen 20 zur Aufnahme von Applikatoren mit stehend nach oben gerichteten Halteschäften versehen ist und gleichzeitig auch einen Haltegriff 1 aufnimmt. Nach Abziehen der Abdeckfolie kann zunächst der Haltegriff 1 entnommen werden, um an einen der nach oben gerichteten Halteschäfte eines Applikators angesetzt zu werden und diesen aus seinem Näpfchen 20 herauszu ziehen. Gegebenenfalls sind die Näpfchen ihrerseits nochmals durch eine durchsichtige Folie so abgedeckt, daß sie einzeln freigelegt werden können, so daß beim Herausnehmen des Stils oder eines Applikators alle anderen noch vollständig luft- und feuchtigkeitsdicht verpackt bleiben können.

Die Fig. 17 zeigt schließlich eine Blisterstreifen 22 zwischen dessen zwei Folien randseitig die Applikatorköpfe 7 einer Vielzahl von Applikatoren 6 hygienisch verpackt untergebracht sind, während die Halteschäfte 5 über die Kanten 23 des Blisterstreifens nach außen überstehen. An diese Halteschäfte 5 kann somit ein Haltegriff angesetzt werden, um anschließend den Applikatorkopf quasi zwischen den Folien der Blisterverpackung herauszureißen.

## Ansprüche

1. Einmal-Applikationsgerät mit einem den Halteschaft eines Wegwerf-Applikators aufnehmenden stilförmigen Haltegriff, mit vorzugsweise abgewinkeltem Kopfende, insbesondere für Kosmetikanwendungen, Dentallabors, Zahnärzte od.dgl., dadurch gekennzeichnet, daß der Haltegriff (1, 1', 1") und der Wegwerf-Applikator (6) so ausgebildet sind, daß der Applikator (6) berührungsfrei am Haltegriff (1', 1', 1") befestigt und wieder gelöst werden kann.

2. Applikationsgerät nach Anspruch 1, dadurch gekennzeichnet, daß der Haltegriff (1', 1") zumindest im vorderen Abschnitt gabelförmig derart ausgebildet ist, daß die Gabelarme (14) mit Haltezapfen (15) von außen in, vorzugsweise als Durchgangsbohrungen ausgebildete, Vertiefungen (16) des Halteschafts (5) des Applikators (6), bzw. umgekehrt, eingreifen und daß zur Erzielung einer verkippsicheren Halterung ineinandergreifende Nut- und Federabschnitte des Haltegriffs (1', 1") und des Halteschafts (5) des Wegwerf-Applikators (6) vorgesehen sind.

3. Applikationsgerät nach Anspruch 2, dadurch gekennzeichnet, daß die Gabelarme (14) von außen federnd am Halteschaft (5) des Wegwerf-Applikators (6) anliegen.

4. Applikationsgerät nach Anspruch 2 oder 3, gekennzeichnet durch eine lösbare Verriegelungseinrichtung für die in Klemmstellung gegeneinander verspannten, in Ruhestellung nach außen federnden Gabelarme.

5. Applikationsgerät nach Anspruch 4, dadurch gekennzeichnet, daß die Gabelarme (14) auf der Innenseite mit verriegelnd ineinandersprengbaren Rasten und Gegenrasten versehen sind.

6. Applikationsgerät nach Anspruch 4, gekennzeichnet durch einen längs der nach hinten keilförmig sich erweiternden Gabelschenkel (14) arretierbar verschiebbaren Klemmring od.dgl.

7. Applikationsgerät nach einem der Ansprüche 2 bis 6, dadurch gekennzeichnet, daß der Haltegriff ein im wesentlichen U-förmiger Bügel aus federelastischem Werkstoff ist.

8. Applikationsgerät nach einem der Ansprüche 2 bis 7, dadurch gekennzeichnet, daß die Vertiefungen (16) in zum freien Ende offenen Nuten (17) des Halteschafts (5) des Applikators (6) angeordnet sind, die der Breite der die Haltezapfen (15) tragenden Klemmenden (13) des Haltegriffs (1', 1") entsprechen.

9. Applikationsgerät nach Anspruch 1, dadurch gekennzeichnet, daß das Kopfende (3) des Haltegriffs (1) eine nach oben offene Bohrung (4) zur Aufnahme des Halteschafts (5) des Applikators (6) aufweist, der mit einer vorzugsweise durchgehenden Ausnehmung (8) für einen längs des Haltegriffs (1) verschiebbar gelagerten Arretierschieber (2) versehen ist.

10. Applikationsgerät nach Anspruch 9, dadurch gekennzeichnet, daß der Halteschaft (5) gegenüber der Bohrung (4) verlängert nach oben aus ihr herausragt.

11. Applikationsgerät, insbesondere nach einem der Ansprüche 1 bis 10, dadurch gekennzeichnet, daß eine Vielzahl von Wegwerf-Applikatoren (6) stehend mit nach oben gerichteten Halteschäften (5) in vorzugsweise einzeln freilegbaren Vertiefungen (20) einer Tiefziehfolie (19) gelagert sind.

12. Applikationsgerät, insbesondere nach einem der Ansprüche, 1 bis 10, dadurch gekennzeichnet, daß eine Vielzahl von Wegwerf-Applikatoren (6) mit frei längs einer Kante (23) nach außen ragenden Halteschäften (5) derart in Blisterstreifen (22) verpackt sind, daß die geschützt untergebrachten Applikatorköpfe (7) nach Verbindung mit dem Haltegriff herausziehbar sind.

FIG. 1

FIG. 2

FIG. 3

FIG. 4

FIG. 5

FIG. 6

FIG. 7

FIG. 8

FIG. 9

FIG. 10

FIG. 11

EP 0 327 803 A2

FIG. 12

FIG. 13

FIG. 14

FIG. 15

FIG. 16

FIG. 17